# EUROPÄISCHE PATENTSCHRIFT

(11) **EP 2 009 438 B1**
(45) Veröffentlichungstag und Bekanntmachung des Hinweises auf die Patenterteilung: **20.08.2014**
(21) Anmeldenummer: 07012760.0
(22) Anmeldetag: 29.06.2007
(51) Int. Cl.: G01N 21/03, G01N 33/28, G01N 21/35, G01N 21/05, G01N 21/15, G01N 21/85

(54) **Verfahren und Einrichtung zur Bestimmung des Wassergehalts in Mineralölen und ähnlichen Flüssigkeiten**
Method and device for determining the water content in mineral oils and similar liquids
Procédé et dispositif destinés à la détermination de la teneur en eau d'huiles minérales et de liquides similaires

(43) Veröffentlichungstag der Anmeldung: 31.12.2008
(73) Patentinhaber: Martechnic GmbH, 22459 Hamburg (DE)
(72) Erfinder: Thöne, Ernst, Dipl.-Ing., 22149 Hamburg (DE); Martin, Hans, Dipl.-Ing., 21436 Marschacht (DE)
(74) Vertreter: Meyer, Ludgerus

(56) Entgegenhaltungen:
- EP-A- 1 402 962
- DE-A1- 3 712 879
- US-A- 3 462 596
- US-A- 3 526 127
- US-A- 4 649 711
- US-A- 5 107 118
- US-A- 5 517 427
- US-A1- 2003 164 451
- US-A1- 2006 192 122

## Beschreibung

Die Erfindung betrifft ein Verfahren zur Bestimmung des Wassergehalts in Mineralölen und ähnlichen Flüssigkeiten nach dem Oberbegriff des Anspruchs 1 sowie eine entsprechende Einrichtung nach dem Oberbegriff des Anspruchs 5.

Bei Verbrennungsmaschinen, wie z.B. bei einer Maschinenanlage auf Schiffen, ist es notwendig, den Eintrag von Wasser in das Schmieröl zu verhindern oder so weit wie möglich zu begrenzen. Der Eintritt von Wasser in das Schmieröl kann über Luftfeuchtigkeit oder durch Leckagen von Wasser führenden Kühlrohren erfolgen. Ein zu hoher Anteil von Wasser im Schmieröl führt zu Korrosion und vermindert die Schmierfähigkeit des Schmieröls.

Aufgrund unterschiedlicher Siedetemperaturen von Öl und Wasser treten Probleme von Wasseranteilen im Öl vor allem im Kurzstreckenbetrieb von Schiffsmaschinen auf, insbesondere bei geringer Drehzahl. Bei längeren Stillstandszeiten lagert sich Wasser außerdem in den unteren Bereichen einer Maschinenanlage an und fördert dort die Korrosion. Außerdem können wasserlösliche Motorenölanteile durch Wasser im Schmieröl gebunden werden und die Viskosität des Öls kann verändert werden. Es ist daher wichtig, den Wassergehalt eines Schmieröls ständig zu überprüfen. Dabei ist es erforderlich, eine einfache Messeinrichtung verwenden zu können, da es z.B. auf Schiffen nicht möglich ist, aufwendige Laboranalysen während einer Fahrt durchzuführen.

Generell müssen im Schiffsbetrieb einsetzbare Sensoren äußerst zuverlässig, wenig störanfällig und langlebig sein. Andererseits ist es nicht erforderlich, eine hochgenaue Messung des Wassergehalts durchzuführen, da im Schiffsbetrieb lediglich eine Aussage darüber erforderlich ist, ob die Maschinenanlage Fehler aufweist oder ob der Zustand eines Öls sich so stark verschlechtert hat, dass ein Ölwechsel erforderlich ist. Daher reicht für den vorgesehenen Einsatzzweck eine relativ grobsensorische Erfassung des Wassergehalts eines Öls aus. Gleichwohl ist generell der Wunsch vorhanden, auch mit einfachen Sensoren eine möglichst hohe Aussagegenauigkeit von erfassten Messwerten zu erzielen.

Es ist bekannt, dass der Verschleißgrad eines Öls, insbesondere eines Motoröls, mittels Infrarotspektroskopie bestimmt werden kann. Im mittleren Infrarotbereich finden sich Absorptionsbanden, die charakteristisch für den Säuregehalt, den Alkoholanteil oder den Wassergehalt sind. Insbesondere findet sich im Bereich von 2900 - 3000 nm ein Spektralbereich, der charakteristisch für den Wassergehalt eines Schmieröls ist. Mittels Infrarotdurchleuchtung eines Schmieröls kann daher festgestellt werden, wie hoch der Wasseranteil ist.

Aus der DE 101 19 932 A1 ist ein Transmissionssensor bekannt, der eine Trübungsmessung in einem Fluid durchführen kann. Die festgestellte Trübung eines Öls wird als Maß für den Wassergehalt angenommen. Da im Spektralbereich von ca. 3.000 nm die Trübung eines Öls jedoch nur relativ geringe Bedeutung hat und außerdem von einer Vielzahl anderer Faktoren, beispielsweise der Art des Öls, dem Anteil von Additiven usw. abhängt, ist eine Trübungsmessung nur bedingt verwertbar.

Ein ähnliches Sensorsystem ist aus der DE 199 57 592 A1 bekannt, bei dem ebenfalls die Trübung eines Öls festgestellt wird. Bei großen Verunreinigungen des Öls kann eine Bypassleitung verwendet werden, die einen Absorber zur Reduzierung des Gehalts von Verunreinigungen im Öl aufweist.

Aus der DE 196 50 397 A1 ist ein Verfahren zur Ermittlung des Verschleißgrades von Öl bekannt, bei dem eine Erfassung der Infrarotstrahlung im Bereich von 10,3 µm erfolgt. Um die Messung unabhängig von der Grundtransmission vornehmen zu können, wird ein Vergleich der Absorption im Bereich einer anderen geeigneten Wellenlänge durchgeführt, so dass die Messung der Transmission eine Relativmessung zur Transmission bei einer anderen Wellenlänge ist. Eine Erfassung des Wassergehalts eines Öls ist bei diesem Verfahren nicht vorgesehen.

Schließlich ist aus der DE 37 12 879 A1 ein Verfahren zur Bestimmung des Wassergehalts in Flüssigkeiten auf Ölbasis und in ähnlichen Flüssigkeiten bekannt, bei dem zwei Filterdetektorpaare verwendet sind, von denen das eine nur eine Wellenlänge in einem Bereich durchlässt, in dem die Absorption von Wasser groß ist, und das andere nur eine Wellenlänge in einem Bereich durchlässt, in dem keine wesentliche Absorption von Wasser erfolgt. Damit kann eine Bestimmung des Wassergehalts eines Öls ohne Bedarf einer Referenzflüssigkeit erfolgen. Jedoch können sich im Laufe der Zeit auch die Transmissionseigenschaften in dem Spektralbereich ändern, der als "Referenzspektralwert" verwendet wird, so dass ohne Kenntnis der Eigenschaften eines Referenzöls eine quantitative Bestimmung des Wassergehalts in einem Öl fehlerhaft sein kann.

Der Erfindung liegt die Aufgabe zugrunde, ein Verfahren und eine Einrichtung zur Bestimmung des Wassergehalts in Mineralölen und ähnlichen Flüssigkeiten mittels einer Infrarotmesszelle anzugeben, bei dem bzw. der mit einfachen Mitteln eine kontinuierliche Erfassung des Wassergehalts eines Mineralöls oder einer ähnlichen Flüssigkeit möglich ist, wobei gleichwohl eine hohe Genauigkeit erreichbar ist und ein Langzeitbetrieb gewährleistet ist.

Diese Aufgabe wird durch ein Verfahren gemäß den kennzeichnenden Merkmalen des Anspruchs 1 sowie eine Einrichtung gemäß den kennzeichnenden Merkmalen des Anspruchs 5 gelöst.

Vorteilhafte Weiterbildungen der Erfindung werden in Unteransprüchen angegeben.

Die Erfindung geht aus von einem Verfahren zur Bestimmung des Wassergehalts in Mineralölen und ähnlichen Flüssigkeiten mittels einer Infrarotmesszelle, die kontinuierlich von dem zu untersuchenden Mineralöl durchströmt wird und deren Ausgangswert bei einem gewählten Spektralwert ein Maß für den Wassergehalt des Mineralöls bildet. Erfindungsgemäß wird der Ausgangsmesswert in Beziehung gesetzt zu einem Basiswert eines Referenzmineralöls und ferner wird aus der Basenzahl des zu messenden Mineralöls ein Korrekturwert abgeleitet, mit dem der Ausgangsmesswert zur Ermittlung eines Anzeigewerts des Wassergehalts kalibriert wird.

Der Messwert der Infrarotmesszelle wird daher auf ein Referenzmineralöl bezogen, um so eine absolute Feststellung der Transmissionseigenschaften bei einem bestimmten Spektralwert und damit eine Aussage über den Wassergehalt zu erhalten. Da bei einer hohen Basenzahl des zu messenden Mineralöls eine Abweichung des gemessenen Wertes vom tatsächlichen Wassergehalt verursacht wird, sieht die Erfindung vor, dass zusätzlich ein Korrekturwert verwendet wird, der aus der Basenzahl des gemessenen Mineralöls abgeleitet ist. Mit Hilfe des Korrekturwertes wird der Ausgangsmesswert zur Ermittlung eines Anzeigewerts des tatsächlichen Wassergehalts kalibriert.

Mittels der Erfindung lässt sich daher der tatsächliche Wassergehalt unabhängig von der Basenzahl des zu messenden Mineralöls feststellen. Die apparatemäßige Messung des Wassergehalts erfolgt mit einer Einrichtung gemäß Anspruch 5, welche auch eine Recheneinheit enthält, in der einerseits ein Basiswert aus der Messung eines Referenzmineralöls und andererseits ein Korrekturwert gespeichert ist, der aus der jeweiligen Basenzahl des zu untersuchenden Mineralöls abgeleitet ist. Die Recheneinheit korrigiert damit den Ausgangsmesswert derart, dass sich nach Korrektur der tatsächliche Wassergehalt des zu untersuchenden Öls ergibt.

Vorzugsweise erfolgt die Messung des Basiswerts des Referenzmineralöls in bestimmten zeitlichen Abständen ebenfalls mittels der Infrarotmesszelle. Diese Messung kann in relativ großen zeitlichen Abständen erfolgen, da das Referenzmineralöl sich nicht verändert. Bei einem Ölwechsel oder einer Änderung der Ölart kann jedoch eine Neubestimmung des Basiswerts erfolgen.

Die Ermittlung des Korrekturwerts erfolgt vorzugsweise aus einer in einem Speicher der Recheneinheit niedergelegten Nachschlagetabelle, die für eine jeweilige Basenzahl einen Korrekturwert enthält, mit dem der Ausgangsmesswert durch Addition, Subtraktion oder Multiplikation verknüpft wird. Die Basenzahl wird auf bekannte Weise mittels eines weiteren an sich bekannten Sensors kontinuierlich oder in zeitlichen Abständen ermittelt und bei der Erfindung entsprechend berücksichtigt.

Zur Vermeidung einer Verschmutzung der kontinuierlich betriebenen Infrarotmesszelle ist vorzugsweise vorgesehen, die Messzelle in zeitlichen Abständen mittels einer Spüllösung von Verunreinigungen zu befreien.

Obgleich die Transmissionseigenschaften eines Mineralöls, wie es für Schiffsmaschinen verwendet wird, in weiten Bereichen vom Verschmutzungsgrad des Öls unabhängig sind, kann es für sehr dunkle oder sehr stark verschmutzte Öle vorteilhaft sein, das zu untersuchende Mineralöl in einem festen Verhältnis mit einem unverschmutzten Mineralöl der gleichen Mineralölart oder einem Neutralöl zu verdünnen, um die Transmissionseigenschaften des Öls in der Messzelle zu verbessern. Der Ausgangsmesswert wird dann im Verhältnis des Mischungsgrades angepasst.

Da der Schwärzungsgrad des Mineralöls auch zu einer Änderung des erfassten Wassergehalts gegenüber dem tatsächlichen Wassergehalt führen kann, wird eine für Weißlicht empfindliche Fotozelle verwendet, die die Lichtdurchlässigkeit des zu prüfenden Mineralöls feststellt, und deren Messwert über eine Nachschlagetabelle rechnerisch mit dem gemessenen Wassergehalt kalibriert wird.

Die erfindungsgemäße Einrichtung verwendet eine Infrarotmesszelle, die vorzugsweise im Kreuzungspunkt eines Messkanals und eines Spülkanals angeordnet ist, wobei Spülkanal und Messkanal mittels Ventilen alternativ verschließbar sind. Damit kann nach Bedarf eine kurzzeitige Unterbrechung der Messung des Wassergehalts vorgenommen werden und während dieser Zeit eine Spülung durch den Spülkanal erfolgen.

Um den Druck in der Infrarotmesszelle zu begrenzen, kann eine Druckhalteeinrichtung vorgesehen sein. Ferner kann vorgesehen sein, die Temperatur in der Messzelle auf einen bestimmten Wert einzustellen, insbesondere mittels einer Heiz- oder Kühleinrichtung, oder alternativ die Temperatur über eine Korrekturtabelle bei der Messung zu berücksichtigen, wobei sich die Messtemperatur zwischen etwa 20 und 55 Grad Celsius bewegen kann.

Bei stark verschmutzten Ölen ist vorzugsweise eine Mischeinrichtung vorgesehen, mit deren Hilfe das zu untersuchende Mineralöl in einem festen Volumenstromanteil mit einem unverschmutzten Mineralöl oder Neutralöl verdünnbar ist.

Zur Weiterverarbeitung des Ausgangsmesswerts kann dieser und/oder der korrigierte und kalibrierte Messwert zu einer Anzeige und/oder der Speicherung überführt werden.

Die Untersuchung des Mineralöls erfolgt vorzugsweise bei einem Spektralwert von 2700 - 3100 nm, vorzugsweise im Bereich 2900 - 3000 nm. Gegebenenfalls kann zusätzlich eine Untersuchung bei weiteren Spektrallinien vorgenommen werden, die ebenfalls für Wasser empfindlich sind.

Das erfindungsgemäße Verfahren und die erfindungsgemäße Einrichtung sind grundsätzlich nicht nur für Mineralöle, sondern auch für andere Flüssigkeiten verwendbar. Ferner kann die Einrichtung auch für andere Spektrallinien eingesetzt werden, die eine Empfindlichkeit für andere Stoffe als Wasser aufweisen, um so eine Feststellung des Vorhandenseins von anderen interessierenden Stoffen durchführen zu können. Eine bevorzugte Anwendung besteht jedoch in der Feststellung des Wassergehalts von Schmierölen in Schiffsbetriebsmaschinen.

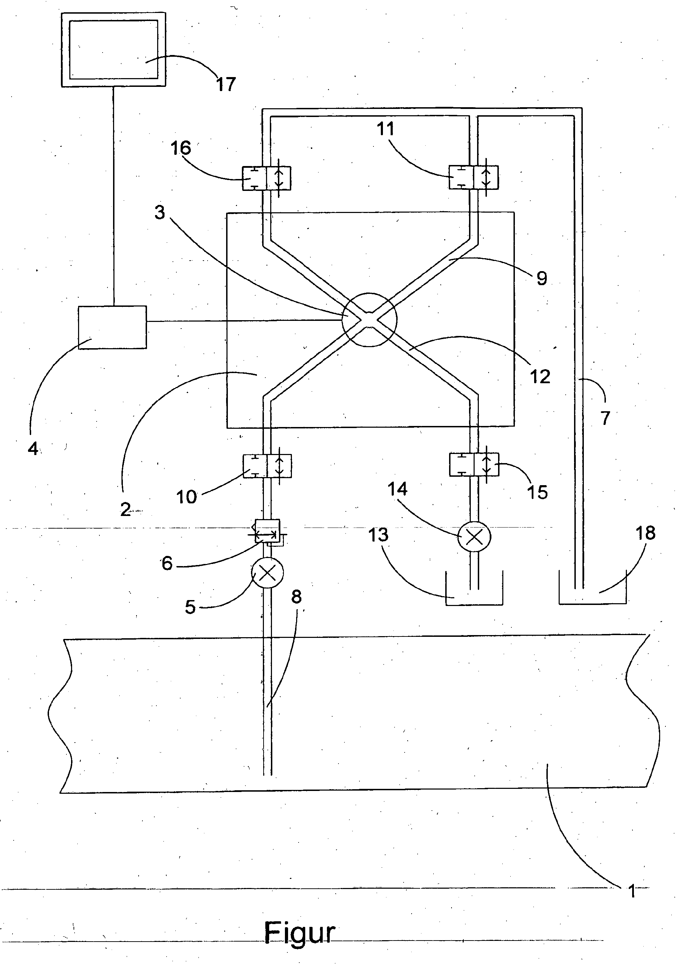

Die Erfindung wird nachstehend anhand eines Ausführungsbeispiels und der beigefügten Zeichnung näher erläutert.

Die einzige Figur zeigt einen Ölbehälter 1, der auch eine Ölleitung sein kann, die Teil einer Schiffsantriebsmaschine ist und das verwendete Schmieröl aufnimmt. In den Behälter 1 taucht eine Ansaugleitung 8 ein, über die mittels einer Pumpe 5 kontinuierlich Mineralöl in kleinen Mengen abgesaugt werden kann. Mittels eines nachgeschalteten Druckhalteventils 6 lässt sich der Pumpdruck beschränken. Über ein Absperrventil 10 wird die angesaugte Flüssigkeit in den Messkanal 9 geleitet und über das Absperrventil 11 in die Ablaufleitung 7 überführt, die in einem drucklosen Auffangbehälter 18 endet.

Der Messkanal 9 verläuft im Ausführungsbeispiel der Messanlage 2 durch zwei parallele Platten, in denen der Kanal 9 eingefräst ist. Die Messanlage 2 nimmt ferner einen Spülkanal 12 auf, wobei Messkanal 9 und Spülkanal 12 sich im Zentrum der Messanlage 2 kreuzen. Die den Spülkanal 12 durchlaufende Flüssigkeit wird aus einer Spülwanne 13 mittels einer Pumpe 14 über ein Absperrventil 15 geleitet und am anderen Ende des Spülkanals 12 über ein Absperrventil 16 ebenfalls an die Ablaufleitung 7 überführt.

Spülkanal 12 und Messkanal 9 sind alternativ über die Absperrventile 10, 11, 15, 16 absperrbar. Zusammen mit der Sperrung der Ventile erfolgt auch eine Umschaltung bzw. Einschaltung der jeweiligen Pumpen 5 bzw. 14.

Im Zentrum der Messanlage 2, d.h. im Kreuzungspunkt zwischen Messkanal 9 und Spülkanal 12, befindet sich eine Infrarotmesszelle 3, die aus einem Infrarotsender und einem Infrarotempfänger besteht, die bezüglich Mess- bzw. Spülkanal gegenüberliegend angeordnet sind. Der Infrarotsender durchleuchtet den Kreuzungspunkt zwischen Mess- und Spülkanal. Auf der gegenüberliegenden Seite der Kanäle ist der Infrarotempfänger angeordnet, der das durch die Flüssigkeit hindurchtretende Infrarotlicht empfängt. Die Infrarotmesszelle kann entweder auf die gewünschte Spektrallinie unmittelbar abgestimmt sein, es können jedoch auch geeignete Filter verwendet werden, die nur die jeweils interessierende Spektrallinie im Bereich von 2900 - 3000 nm zur Feststellung von Wasser, hindurch treten lassen. Der Messpunkt ist als Druckstromküvette ausgebildet mit einer Breite von ca. 0,1 - 0,5 mm. Das Ausgangssignal der Infrarotmesszelle 3 wird auf eine Recheneinheit 4 geleitet, in der der Ausgangsmesswert verarbeitet wird. Insbesondere erfolgt dort eine Bezugnahme auf einen Basiswert eines Referenzmineralöls mit festgestelltem oder mittels der Messanlage 2 ermitteltem Referenzwert. Dadurch lässt sich die Abweichung des erfassten Messwertes von dem für das Referenzmineralöl festgestellten Messwert erfassen. Die Abweichung kann in absoluten Werten oder auch prozentual ausgegeben werden.

Die Recheneinheit 4 enthält ferner einen Speicher, in dem Korrekturwerte gespeichert sind, die der jeweiligen Basenzahl des erfassten Mineralöls entsprechen. Die Basenzahl kann entweder manuell erfasst und in der Recheneinheit gespeichert werden oder sie kann kontinuierlich mittels einer nicht weiter dargestellten Sensoreinrichtung erfasst und kontinuierlich in die Recheneinheit 4 eingeführt werden. Der von der Infrarotmesszelle 3 erhaltene Messwert wird auf diese Weise auf die Basenzahl kalibriert, so dass die Einflussnahme der Basenzahl, die anderenfalls zu einer Verfälschung des Messergebnisses führen würde, ausgeglichen werden kann. Damit kann der tatsächliche Wassergehalt des Mineralöls festgestellt werden, ohne dass die Basenzahl Einfluss auf das Messergebnis nimmt.

Kleine Basenzahlen unter 10 erfordern in der Regel keine Kalibrierung. Bei Werten über 10 sollte jedoch eine Kalibrierung des Ausgangsmesswertes in Abhängigkeit von der Basenzahl vorgenommen werden.

Die von der Recheneinheit 4 ermittelten Werte können dann entweder an einer Anzeigeeinheit 17 zur Anzeige kommen, sie können jedoch auch in einer weiteren Recheneinheit gespeichert, an Steuerstände in einem Schiff übermittelt oder auf sonstige Weise per Datenfernverarbeitung an relevante Stellen übermittelt werden. Die Messwerte können auch an automatische Überwachungseinrichtungen übermittelt werden, so dass bei plötzlichem Anstieg des Wassergehalts im Mineralöl entsprechende Warnmeldungen ausgegeben werden, die beispielsweise einen Maschinenstopp auslösen können.

Die Einstellung des Drucks des zu messenden Mineralöls ist unkritisch, jedoch sollte für eine ausreichende Funktion der Einrichtung der Druck auf ca. 1 bar begrenzt werden. Als Messtemperatur sollte ein Wert von 80°C nicht überschritten werden.

Für den Fall, dass eine Messung eines besonders stark verschmutzten Öls bzw. eines sehr dunklen Öls, das für Infrarotlicht praktisch nicht mehr durchlässig ist, vorgenommen werden soll, kann das zu untersuchende Öl zweckmäßigerweise mittels einer in der Zeichnung nicht dargestellten Mischvorrichtung verdünnt werden, indem ein fester Teil eines unverbrauchten Öls oder eines Neutralöls, wie z.B. einem Transformatorenöl, dem zu messenden Öl beigemischt wird. Entsprechend muss das Messergebnis mit einem Korrekturwert belegt werden, um den tatsächlichen Wassergehalt des zu untersuchenden Öls festzustellen.

Die erfindungsgemäße Einrichtung ermöglicht die Erfassung eines Wassergehalts in Schmieröl bis ca. 1 Vol.-%.

### Bezugszeichen

- 1: Ölbehälter
- 2: Messanlage
- 3: Infrarotmesszelle
- 4: Recheneinheit
- 5: Pumpe
- 6: Druckhalteventil
- 7: Ablaufleitung
- 8: Ansaugleitung
- 9: Messkanal
- 10: Absperrventil
- 11: Absperrventil
- 12: Spülkanal
- 13: Spülwanne
- 14: Pumpe
- 15: Absperrventil
- 16: Absperrventil
- 17: Display
- 18: Auffangbehälter

## Patentansprüche

1. Verfahren zur Bestimmung des Wassergehalts in Mineralölen und ähnlichen Flüssigkeiten mit einer bestimmten Basenzahl und einem bestimmten Schwärzungsgrad mittels einer Infrarotmesszelle (3), die in einem Messkanal (9) kontinuierlich von dem zu untersuchenden Mineralöl durchströmt wird und deren Ausgangsmesswert bei einem gewählten Spektralwert ein Maß für den Wassergehalt des Mineralöls bildet, **dadurch gekennzeichnet,**
**dass** der Ausgangsmesswert in Beziehung gesetzt wird zu einem Basiswert eines Referenzmineralöls, wobei im Wechsel zur Messung des zu untersuchenden Mineralöls mittels der Infrarotmesszelle (3) und in bestimmten zeitlichen Abständen der Basiswert des durch einen Spülkanal (12) strömenden Referenzmineralöls ermittelt wird,
**dass** aus der Basenzahl des zu untersuchenden Mineralöls ein erster Korrekturwert abgeleitet wird, mit dem der Ausgangsmesswert der Infrarotmesszelle (3) zur Ermittlung eines Anzeigewerts des Wassergehalts kalibriert wird,
und **dass** der Schwärzungsgrad des zu untersuchenden Mineralöls über einen von dem Grad der Schwärzung abhängigen zweiten Korrekturwert berücksichtigt wird, mit dem der gemessene Wassergehalt rechnerisch kalibriert wird.

2. Verfahren nach Anspruch 1, **dadurch gekennzeichnet, dass** der erste Korrekturwert mittels einer Nachschlagetabelle aus der Basenzahl des zu untersuchenden Mineralöls abgeleitet wird.

3. Verfahren nach Anspruch 1, **dadurch gekennzeichnet, dass** die Infrarotmesszelle (3) in bestimmten zeitlichen Abständen mittels einer Spüllösung von Verunreinigungen befreit wird.

4. Verfahren nach Anspruch 1, **dadurch gekennzeichnet, dass** für starke Verschmutzungen des zu untersuchenden Mineralöls das die Infrarotmesszelle durchströmende Mineralöl in einem festen Verhältnis mit einem unverschmutzten Mineralöl der gleichen Mineralölart verdünnt wird.

5. Einrichtung zur Bestimmung des Wassergehalts in Mineralölen und ähnlichen Flüssigkeiten mit einer bestimmten Basenzahl und einem bestimmten Schwärzungsgrad mittels einer Infrarotmesszelle (3), die in einem Messkanal (9) kontinuierlich von dem zu untersuchenden Mineralöl durchströmt wird und deren Ausgangsmesswert bei einem gewählten Spektralwert ein Maß für den Wassergehalt des Mineralöls bildet, wobei die Infrarotmesszelle (3) als Durchströmküvette ausgebildet ist, bei der ein die Küvette durchleuchtender Infrarotstrahl von einem Infrarotempfänger erfasst wird, **dadurch gekennzeichnet,**
**dass** der Ausgangsmesswert der Infrarotmesszelle (3) mittels einer Recheneinheit (4) in Beziehung gesetzt wird zu einem gespeicherten Basiswert, der im Wechsel zur Messung des zu untersuchenden Mineralöls mittels der Infrarotmesszelle (3) und in bestimmten zeitlichen Abständen aus der Messung eines durch einen Spülkanal (12) strömenden Referenzmineralöls erhalten wurde,
**dass** der Ausgangsmesswert mittels der Recheneinheit ferner mittels eines ersten Korrekturwerts kalibriert wird, der aus der jeweiligen Basenzahl des zu untersuchenden Mineralöls abgeleitet ist,
**dass** ein für Weißlicht empfindlicher Fotoempfänger zur Erfassung des Schwärzungsgrads des zu untersuchenden Mineralöls vorgesehen ist, und
**dass** aus dem erfassten Schwärzungsgrad ein zweiter Korrekturwert abgeleitet wird, mit dessen Hilfe der erfasste Wassergehalt des zu untersuchenden Mineralöls rechnerisch verknüpft wird.

6. Einrichtung nach Anspruch 5, **dadurch gekennzeichnet, dass** der von der Basenzahl abhängige erste Korrekturwert in einer Nachschlagetabelle der Recheneinheit gespeichert ist.

7. Einrichtung nach Anspruch 5, **dadurch gekennzeichnet, dass** die Infrarotmesszelle (3) im Kreuzungspunkt des Messkanals (9) und des Spülkanals (12) angeordnet ist, wobei Spülkanal (12) und Messkanal (9) mittels Ventilen (10, 11; 15, 16) alternativ verschließbar sind.

8. Einrichtung nach Anspruch 5, **dadurch gekennzeichnet, dass** im Messkanal (3) eine Druckhalteeinrichtung (6) zur Festlegung des Messdrucks im Messkanal (9) vorgesehen ist.

9. Einrichtung nach Anspruch 5, **dadurch gekennzeichnet, dass** eine Temperatureinstelleinrichtung zur Einstellung der Messtemperatur in der Infrarotmesszelle (3) vorgesehen ist.

10. Einrichtung nach Anspruch 5, **dadurch gekennzeichnet, dass** ein Temperaturfühler zur Erfassung der Messtemperatur des zu untersuchenden Mineralöls vorgesehen ist, und dass der gemessene Wassergehalt des zu untersuchenden Mineralöls mit einem der erfassten Messtemperatur zugeordneten dritten Korrekturwert hard- oder softwaremäßig kalibriert wird.

11. Einrichtung nach Anspruch 5, **dadurch gekennzeichnet, dass** eine Mischeinrichtung vorgesehen ist, mit deren Hilfe das zu untersuchende Mineralöl in einem festen Verhältnis mit einem Volumenstrom eines unverschmutzten Mineralöls verdünnbar ist.

12. Einrichtung nach Anspruch 10, **dadurch gekennzeichnet, dass** mittels einer Nachschlagetabelle der erfasste Messwert des Wassergehalts rechnerisch mit einem jeweils einer Messtemperatur zugeordneten dritten Korrekturwert kalibriert wird.

13. Einrichtung nach Anspruch 5, **dadurch gekennzeichnet, dass** der Ausgangsmesswert und/oder der korrigierte und kalibrierte Messwert an eine Anzeige- und/oder Speichereinheit (17) geführt wird.

14. Einrichtung nach Anspruch 5, **dadurch gekennzeichnet, dass** der gewählte Spektralwert 2900 - 3000 nm beträgt.

## Claims

1. Method for determining the water content in mineral oils and similar liquids having a specific base number and a specific degree of blackening, by means of an infra-red measuring cell (3) through which the mineral oil to be tested flows continuously in a measuring channel (9) and the output measurement of which at a chosen spectral value constitutes a measure of the water content of the mineral oil, **characterised in that** the output measurement is related to a base value of a reference mineral oil, the base value of the reference mineral oil flowing through a flushing channel (12) being determined in alternation with the measurement of the mineral oil to be tested by means of the infra-red measuring cell (3) and at specific time intervals,
**in that** a first correction value is derived from the base number of the mineral oil to be tested, with which first correction value the output measurement of the infra-red measuring cell (3) is calibrated to determine a display value of the water content,
and **in that** the degree of blackening of the mineral oil to be tested is taken into account via a second correction value which is dependent on the degree of blackening, with which second correction value the measured water content is calibrated mathematically.

2. Method according to claim 1, **characterised in that** the first correction value is derived by means of a look-up table from the base number of the mineral oil to be tested.

3. Method according to claim 1, **characterised in that** the infra-red measuring cell (3) is freed of impurities at specific time intervals by means of a flushing solution.

4. Method according to claim 1, **characterised in that**, for heavy contamination of the mineral oil to be tested, the mineral oil flowing through the infra-red measuring cell is diluted in a fixed proportion with an uncontaminated mineral oil of the same mineral oil type.

5. Device for determining the water content in mineral oils and similar liquids having a specific base number and a specific degree of blackening, by means of an infra-red measuring cell (3) through which the mineral oil to be tested flows continuously in a measuring channel (9) and the output measurement of which at a chosen spectral value constitutes a measure of the water content of the mineral oil, the infra-red measuring cell (3) being in the form of a flow-through cuvette in which an infra-red beam shining through the cuvette is detected by an infra-red receiver,
**characterised in that**
the output measurement of the infra-red measuring cell (3) is related, by means of a processing unit (4), to a stored base value which was obtained in alternation with the measurement of the mineral oil to be tested by means of the infra-red measuring cell (3) and at specific time intervals from the measurement of a reference mineral oil flowing through a flushing channel (12),
**in that** the output measurement is further calibrated by means of the processing unit by means of a first correction value which is derived from the base number of the mineral oil to be tested,
**in that** a photoreceiver that is sensitive for white light is provided for determining the degree of blackness of the mineral oil to be tested, and
**in that** a second correction value is derived from the determined degree of blackness, by means of which second correction value the determined water content of the mineral oil to be tested is linked mathematically.

6. Device according to claim 5, **characterised in that** the first correction value, which is dependent on the base number, is stored in a look-up table of the processing unit.

7. Device according to claim 5, **characterised in that** the infra-red measuring cell (3) is arranged at the point of intersection of the measuring channel (9) and the flushing channel (12), it being possible for the flushing channel (12) and the measuring channel (9) to be closed alternately by means of valves (10, 11; 15, 16).

8. Device according to claim 5, **characterised in that** a pressure-maintaining device (6) is provided in the measuring channel (3) for setting the measuring pressure in the measuring channel (9).

9. Device according to claim 5, **characterised in that** a temperature-adjusting device is provided for adjusting the measuring temperature in the infra-red measuring cell (3).

10. Device according to claim 5, **characterised in that** a temperature sensor is provided for determining the measuring temperature of the mineral oil to be tested, and **in that** the measured water content of the mineral oil to be tested is calibrated by means of hardware or software with a third correction value associated with the determined measuring temperature.

11. Device according to claim 5, **characterised in that** a mixing device is provided, by means of which the mineral oil to be tested can be diluted in a fixed proportion with a volume flow of an uncontaminated mineral oil.

12. Device according to claim 10, **characterised in that**, by means of a look-up table, the determined measurement of the water content is calibrated mathematically with a third correction value associated with a measuring temperature.

13. Device according to claim 5, **characterised in that** the output measurement and/or the corrected and calibrated measurement is fed to a display and/or storage unit (17).

14. Device according to claim 5, **characterised in that** the chosen spectral value is from 2900 to 3000 nm.

## Revendications

1. Procédé pour déterminer la teneur en eau d'huiles minérales et de liquides similaires avec un indice de basicité déterminé et un degré de noircissement déterminé, à l'aide d'une cellule de mesure infrarouge (3) qui est traversée de manière continue dans un canal de mesure (9) par l'écoulement de l'huile minérale à analyser, et dont la valeur de mesure de sortie, avec une valeur spectrale choisie, forme une mesure pour la teneur en eau de l'huile minérale, **caractérisé**
**en ce que** la valeur de mesure de sortie est mise en relation avec une valeur de base d'une huile minérale de référence, étant précisé qu'en alternance avec la mesure de l'huile minérale à analyser, à l'aide de la cellule de mesure infrarouge (3) et suivant une périodicité déterminée, la valeur de base de l'huile minérale de référence qui traverse un canal à écoulement rapide (12) est recherchée,
**en ce qu'**à partir de l'indice de basicité de l'huile minérale à analyser est dérivée une première valeur de correction avec laquelle la valeur de mesure de sortie de la cellule de mesure infrarouge (3) est étalonnée pour rechercher une valeur d'indication de la teneur en eau,
et **en ce que** le degré de noircissement de l'huile minérale à analyser est pris en compte par l'intermédiaire d'une deuxième valeur de correction, dépendante du degré de noircissement, avec laquelle la teneur en eau mesurée est étalonnée par calcul.

2. Procédé selon la revendication 1, **caractérisé en ce que** la première valeur de correction est dérivée, à l'aide d'une table de consultation, de l'indice de basicité de l'huile minérale à analyser.

3. Procédé selon la revendication 1, **caractérisé en ce que** la cellule de mesure infrarouge (3) est débarrassée des impuretés, suivant une périodicité déterminée, à l'aide d'une solution de rinçage.

4. Procédé selon la revendication 1, **caractérisé en ce que** pour de forts encrassements de l'huile minérale à analyser, l'huile minérale qui traverse la cellule de mesure infrarouge est diluée suivant un rapport fixe avec une huile minérale, non encrassée, du même type d'huile minérale.

5. Dispositif pour déterminer la teneur en eau d'huiles minérales et de liquides similaires avec un indice de basicité déterminé et un degré de noircissement déterminé, à l'aide d'une cellule de mesure infrarouge (3) qui est traversée de manière continue dans un canal de mesure (9) par l'écoulement de l'huile minérale à analyser, et dont la valeur de mesure de sortie, avec une valeur spectrale choisie, forme une mesure pour la teneur en eau de l'huile minérale, étant précisé que la cellule de mesure infrarouge (3) est conçue comme une cuvette de passage, un rayon infrarouge qui traverse ladite cuvette étant détecté par un récepteur infrarouge, **caractérisé**
**en ce que** la valeur de mesure de sortie de la cellule de mesure infrarouge (3) est mise en relation, à l'aide d'une unité de calcul (4), avec une valeur de base stockée qui, en alternance avec la mesure de l'huile minérale à analyser, à l'aide de la cellule de mesure infrarouge (3) et suivant une périodicité déterminée, a été obtenue à partir de la mesure d'une huile minérale de référence qui traverse un canal à écoulement rapide (12),
**en ce que** la valeur de mesure de sortie est également étalonnée à l'aide de l'unité de calcul à l'aide d'une première valeur de correction qui est dérivée de l'indice de basicité de l'huile minérale à analyser,
**en ce qu'**il est prévu un photorécepteur sensible à la lumière blanche, pour détecter le degré de noircissement de l'huile minérale à analyser, et
**en ce qu'**une deuxième valeur de correction est dérivée du degré de noircissement détecté, à l'aide de laquelle la teneur en eau détectée de l'huile minérale à analyser est combinée par calcul.

6. Dispositif selon la revendication 5, **caractérisé en ce que** la première valeur de correction, dépendante de l'indice de basicité, est stockée dans une table de consultation de l'unité de calcul.

7. Dispositif selon la revendication 5, **caractérisé en ce que** la cellule de mesure infrarouge (3) est disposée à l'intersection du canal de mesure (9) et du canal d'écoulement rapide (12), étant précisé que le canal à écoulement rapide (12) et le canal de mesure (9) sont aptes à être fermés en alternance à l'aide de soupapes (10, 11 ; 15, 16).

8. Dispositif selon la revendication 5, **caractérisé en ce qu'**il est prévu dans le canal de mesure (3) un dispositif de maintien de pression (6) pour fixer la pression de mesure dans le canal de mesure (9)

9. Dispositif selon la revendication 5, **caractérisé en ce qu'**il est prévu un dispositif de réglage de température pour régler la température dans la cellule de mesure infrarouge (3).

10. Dispositif selon la revendication 5, **caractérisé en ce qu'**il est prévu un capteur de température pour détecter la température de mesure de l'huile minérale à analyser, et **en ce que** la teneur en eau mesurée de l'huile minérale à analyser est étalonnée par matériel ou logiciel avec une troisième valeur de correction affectée à la température de mesure détectée.

11. Dispositif selon la revendication 5, **caractérisé en ce qu'**il est prévu un dispositif mélangeur à l'aide duquel l'huile minérale à analyser peut être diluée suivant un rapport fixe avec un flux volumique d'une huile minérale non encrassée.

12. Dispositif selon la revendication 10, **caractérisé en ce que**, à l'aide d'une table de consultation, la valeur de mesure de la teneur en eau est étalonnée par calcul avec une troisième valeur de correction affectée à une température de mesure.

13. Dispositif selon la revendication 5, **caractérisé en ce que** la valeur de mesure de sortie et/ou la valeur de mesure corrigée et étalonnée sont transmises à une unité d'affichage et/ou de stockage (17).

14. Dispositif selon la revendication 5, **caractérisé en ce que** la valeur spectrale choisie est de 2900-3000 nm.
